# EUROPEAN PATENT SPECIFICATION

(11) **EP 3 192 496 B1**
(45) Date of publication and mention of the grant of the patent: **24.08.2022**
(21) Application number: 17150848.4
(22) Date of filing: 10.01.2017
(51) Int. Cl.: A61K 8/64, A61K 8/66, A61Q 11/00, A61K 38/44, A61K 38/46, A61K 38/47, A61K 38/54, A61P 1/02, A61K 38/40, A61K 9/00, A61K 47/36

(54) **ORAL CARE COMPOSITIONS**
MUNDPFLEGEZUSAMMENSETZUNGEN
COMPOSITIONS DE PROTECTION ORALE

(30) Priority: 12.01.2016 EP 16150971; 27.06.2016 EP 16176459; 27.06.2016 EP 16176464; 30.06.2016 EP 16177374
(43) Date of publication of application: 19.07.2017
(73) Proprietor: Unilever IP Holdings B.V., 3013 AL Rotterdam (NL); Unilever Global IP Limited, Wirral, Merseyside CH62 4ZD (GB)
(72) Inventor: ADAMS, Suzanne, Elizabeth, Bebington, Wirral, Merseyside CH63 3JW (GB); ARNOLD, David, Stanley, Bebington, Wirral, Merseyside CH63 3JW (GB); BRADING, Melanie, Gayle, Bebington, Wirral, Merseyside CH63 3JW (GB); GREEN, Alison, Katharine, Bebington, Wirral, Merseyside CH63 3JW (GB); MURPHY, Barry, Bebington, Wirral, Merseyside CH63 3JW (GB)
(74) Representative: Tansley, Sally Elizabeth

(56) References cited:
- CN-A- 101 721 324
- JP-A- 2002 322 088
- KR-A- 20030 003 477
- RU-A- 2012 101 121
- RU-C2- 2 496 468
- US-A1- 2009 324 547
- F. E. DEWHIRST ET AL: "The Human Oral Microbiome", JOURNAL OF BACTERIOLOGY, vol. 192, no. 19, 1 October 2010 (2010-10-01), pages 5002-5017, XP055289194, US ISSN: 0021-9193, DOI: 10.1128/JB.00542-10
- WONG ET AL: "Pathogenic ''nonpathogenic'' Neisseria spp", CLINICAL MICROBIOLOGY NEWSLETTER, ELSEVIER, NEW YORK, NY, US, vol. 16, no. 6, 15 March 1994 (1994-03-15) , pages 41-44, XP023706284, ISSN: 0196-4399, DOI: 10.1016/0196-4399(94)90026-4 [retrieved on 1994-03-15]

## Description

### Field of the Invention

The present invention is concerned with oral care compositions. More particularly, the present invention is concerned with oral care compositions containing proteins.

### Background of the Invention

Conventional thinking with respect to toothpastes is that anti-bacterials are needed to destroy the bacteria within the mouth. KR 2003 0003477, JP 2002 322088 and US 2009/324547 disclose oral care compositions comprising a mixture of enzymes with antibacterial activity. The present application has found that it is beneficial to adjust the balance away from bacteria associated with poor oral health in favour of those associated with good oral health. There is therefore a need for a composition that increases the level of health associated-bacteria in the mouth.

The present application relates to compositions that increase the prominence of beneficial bacterial flora in the mouth.

### Description of the Invention

The present application relates to compositions comprising proteins for use in a method to increase the health related bacterial flora of the mouth.

### Detailed Description of the Invention

The oral microbiome is highly complex and diverse community with over 700 different species having been identified. In general, health-related bacteria are facultative anaerobic or aerobic bacteria (e.g. genus *Neisseria*) and bacteria related to gum disease are anaerobic bacteria (e.g genus *Treponema*)*.* There is therefore a need for the promotion these beneficial bacteria in the mouth.

The present application has found that compositions comprising enzymes can be used to increase the beneficial bacterial of the mouth. The beneficial bacteria are of the genus *Neisseria* and *Prevotella,* and of the species *Neisseria flava, Neisseria (HOT499*|*sp._str._SMC_A9199), Neisseria sp._Oral_Taxon_020* and *Neisseria* sp._str._SMC_A9199, *Prevotella melaninogenica..* Other preferred beneficial bacterial species include *Streptococcus* (*mitis*|*mitis*_bv_2|*oralis*|*pneumoniae*|HOT058|HOT064|HOT070|sp._str._C300), *Lactobacillus gasseri Streptococcu*(*infantis*|*mitis*|*mitis*_bv_2|*pneumoniae*|HOT064).

The present application has found that compositions comprising proteins can be used to increase the numbers of beneficial bacterial in the mouth.

Preferably compositions of the invention comprise lactoferrin, glycoside hydrolase or mixtures thereof.

Preferably the glycoside hydrolase is lysozyme.

The level of lactoferrin in the invention is preferably from 0.01 wt% to 0.1 wt%, more preferably from 0.005 wt% to 0.05 wt%. Other proteins preferably lysozymes, and colostrum may be present in the invention. Preferably these proteins are present at total levels from 0.001 to 0.5 wt% of the total composition, preferably from 0.005 to 0.1 wt% of the total composition.

Preferably the compositions of the invention comprise enzymes. Enzymes are preferably selected from the following groups:
i) an glycoside hydrolase, more preferably an amylase, most preferably amylglucosidase;
ii) an oxoreductase acting on OH groups of donors, more preferably an oxidase with oxygen as acceptor, most preferably glucose oxidase;
iii) a hemeperoxidase more preferably a haloperoxidase, most preferably a lactoperoxidase;
iv) It is particularly preferred if the enzyme is a mixture of the 3 groups above, most preferably a mixture of amylglucosidase, glucose oxidase and lactoperoxidase.

Preferably the total level of enzyme is from 0.01 wt% to 2wt% of the total composition. More preferably from 0.1 wt% to 1 wt% of the total composition.

Preferably the total level of glycoside hydrolase in the composition is from 0.05 to 1 wt% of the total composition.

Preferably the total level of oxoreductase acting on OH groups of donors is from 0.05 to 1 wt% of the total composition.

Preferably the total level of hemeperoxidase is from 0.0005 to 0.01 wt% of the total composition.

Preferably the weight ratio of glycoside hydrolase to other enzymes in the composition is greater than 1:1, more preferably greater than 3:2.

Furthermore, the oral care composition will usually contain a surfactant in an amount of from 0.2 to 5% by weight based on the total weight of the dentifrice. Preferred are nonionic surfactants such as optionally polyethoxylated fatty acid sorbitan esters, ethoxylated fatty acids, esters of polyethylene glycol, ethoxylates of fatty acid monoglycerides and diglycerides, and ethylene oxide/propylene oxide block polymers. Particularly preferred are polyethylene glycol ethers of fatty alcohols in particular polyethylene glycol ethers having from 20 to 40 ethylene oxide groups per unit. An example of such a suitable surfactant includes the group of surfactants known as Steareth surfactants such as Steareth 30. Preferably the level of non-ionic surfactant is from 0.5 to 7 wt% of the total composition, more preferably from 1 to 5 wt% of the total composition

Although other surfactants may be present it is preferred if they are limited to levels below 0.5 wt% of the total composition preferably less than 0.1 wt% of the total composition.

The oral care composition, especially in the cases of dentifrices, dentifrice may also contain structurants and agents such as binders and thickening agents. These structurants will generally be present in an amount of from 0.1 to 1% by weight based on the total weight of the dentifrice. Suitable binders or thickening agents include carboxyvinyl polymers (such as polyacrylic acids cross-linked with polyallyl sucrose or polyallyl pentaerythritol), hydroxyethyl cellulose, hydroxypropyl cellulose, water soluble salts of cellulose ethers (such as sodium carboxymethyl cellulose and sodium carboxymethyl hydroxyethyl cellulose), natural gums (such as carrageenan, gum karaya, guar gum, xanthan gum, gum arabic, and gum tragacanth). Natural gum based thickeners, in particular carrageenan are particularly preferred.

Examples of suitable product forms for compositions of the invention include dentifrices, mouthwashes, mouthsprays and liquid formulations.

Preferred product forms for compositions of the invention are those which are suitable for brushing and/or rinsing the surfaces of the oral cavity. Dentifrices/toothpastes are preferred.

In such preferred product forms, the amount of water and/or polyhydric alcohol will generally be at least 10 percent, preferably at least 30 percent, more preferably at least 50 percent by total weight water and/or polyhydric alcohol based on the total weight of the composition. Preferably the level of water and/or polyhydric alcohol will be less than 90 wt% of the total composition, more preferably less than 85 wt%.

An example of a preferred type of product form in the context of the present invention is a dentifrice. The term "dentifrice" generally denotes formulations which are used to clean the surfaces of the oral cavity. The dentifrice is an oral composition that is not intentionally swallowed for purposes of systemic administration of therapeutic agents, but is applied to the oral cavity, used to treat the oral cavity and then expectorated. Typically the dentifrice is used in conjunction with a cleaning implement such as a toothbrush, usually by applying it to the bristles of the toothbrush and then brushing the accessible surfaces of the oral cavity. Preferably the dentifrice is in the form of a paste or a gel (or a combination thereof).

A dentifrice composition according to the invention will usually contain, as the aqueous continuous phase, a mixture of water and polyhydric alcohol in various relative amounts, with the amount of water generally ranging from 10 to 50% by weight (based on the total weight of the dentifrice) and the amount of polyhydric alcohol generally ranging from 20 to 60% by weight (based on the total weight of the dentifrice).

Typical polyhydric alcohols for use in the oral care composition, particularly a dentifrice composition according to the invention include humectants such as glycerol, sorbitol, polyethylene glycol, polypropylene glycol, propylene glycol, xylitol (and other edible polyhydric alcohols), hydrogenated partially hydrolyzed polysaccharides and mixtures thereof. Glycerol and sorbitol are preferred, sorbitol being particularly preferred.

The oral care composition, in particular dentifrice compositions comprises abrasive materials. Abrasive materials will generally be present in an amount of from 0.5 to 75%, preferably 3 to 60 by weight based on the total weight of the dentifrice. Suitable abrasive cleaning agents include silica xerogels, hydrogels and aerogels and precipitated particulate silicas; calcium carbonate, dicalcium phosphate, tricalcium phosphate, calcined alumina, sodium and potassium metaphosphate, sodium and potassium pyrophosphates, sodium trimetaphosphate, sodium hexametaphosphate, particulate hydroxyapatite and mixtures thereof. Silicas are particularly preferred.

Compositions of the invention may also comprise thickeners such as finely divided silicas, hectorites, calcium carbonate, colloidal magnesium aluminium silicates and mixtures thereof.

Compositions of the invention may comprise a zinc ion. Preferably the sources of zinc ions are zinc chloride, zinc acetate, zinc gluconate, zinc sulphate, zinc fluoride, zinc citrate, zinc lactate, zinc oxide, zinc monoglycerolate, zinc tartrate, zinc pyrophosphate zinc maleate and mixtures thereof.

Compositions of the present invention may also contain further optional ingredients customary in the art such as fluoride ion sources, anticalculus agents, buffers, flavouring agents, sweetening agents, colouring agents, opacifying agents, preservatives, antisensitivity agents, bleaching and antimicrobial agents.

To clean the surfaces of the oral cavity, the composition according to the invention is topically applied to the oral cavity surfaces to be cleaned.

Preferably the composition according to the invention is topically applied to the oral cavity surfaces to be cleaned and then agitated by brushing and/or rinsing.

The invention will now be illustrated by the following non-limiting Examples:

### Examples

A double-blind, randomised, parallel study, was conducted to collect plaque samples. Subjects brushed twice daily for 14 weeks. Samples of plaque were collected at baseline and at the end of the 14-week brushing period for microbiomics analysis. The data generated from 102 subjects, 52 using Example 1 and 50 using Example A -standard silica SLS toothpaste, completed the microbiomics analysis.

### Example 1

| **Material** | **Amount %W/W** |
|---|---|
| Sorbitol | 28.5 |
| Zinc Gluconate | 0.25 |
| Sodium Fluoride | 0.32 |
| Citric Acid | 0.25 |
| Disodium Phosphate | 0.5 |
| Titanium Dioxide | 0.5 |
| Abrasive silica | 16.0 |
| Thickening silica | 4.3 |
| Carrageenan | 0.7 |
| Glycerin | 5.0 |
| Xanthuan m gum | 1.0 |
| Steareth 30 | 3.0 |
| Amylglucosidase | 0.3 |
| Lactoperoxidase | 0.002 |
| Lysozyme (22%)*** | 0.05 |
| Glucose Oxidase | 0.14 |
| Lactoferrin | 0.01 |
| Colostrum | 0.01 |
| Water and minors | to100.0 |

### Example A

| **Material** | **Amount (%W/W)** |
|---|---|
| Sorbitol | 45 |
| PEG-32 | 5 |
| Monosodium Phosphate | 0.1 |
| Sodium Fluoride | 0.32 |
| Titanium Dioxide | 1 |
| Thickening silica | 7.5 |
| Abrasive silica | 10 |
| Cellulose gum | 0.63 |
| Sodium Lauryl Sulphate | 1.5 |
| Water and minors | To 100 |

### Microbiomics analysis:

Plaque samples were processed in pairs (baseline and 14 weeks) with the first step being DNA extraction. This involved an overnight lysis step followed by bead beating and purification using an automated DNA extraction robot. The resulting DNA was quantified and normalised prior to first round polymerase chain reaction (PCR) amplification performed targeting the V4-V6 region of the 16SrRNA gene. The amplicons were processed through a second round of PCR to incorporate Illumina sequencing adapter sequences containing indexes (i5 and i7) for sample identification. The resulting amplicons were then sequenced on an Illumina MiSeq with 2x300bp paired-end sequencing using v3 chemistry. Following sequencing, all the raw reads were processed simultaneously through a bioinformatics pipeline and classified to the species level against the HOMD database. Statistical analysis was performed on the resulting data sets.

### Results:

Following statistical analysis by comparative testing using Dirichlet Multinomial distribution the following changes were identified:

| **Significant Taxa** | **% Change** | **Association** |
|---|---|---|
| ***Neisseria flava*** | 2.869 | Genus associated with health |
| ***Prevotella melaninogenica*** | 0.393 | Health |
| ***Neisseria* (HOT499\|sp._str._SMC_A9199)** | 0.076 | Genus associated with health |
| ***Streptococcus* (*mitis*\|*mitis*_bv_2\|*oralis*\|*pneumoniae*\|HOT058\|HOT064\| HOT070\|sp._str._C300)** | 0.097 | Health |
| *Prevotella* **(*melaninogenica*\|HOT314)** | 0.036 | Health |
| ***Neisseria* sp._Oral_Taxon_020** | 0.088 | Genus associated with health |
| ***Neisseria* sp._str._SMC_A9199** | 0.001 | Genus associated with health |
| ***Streptococcus* (*infantis*\|*mitis*\|*mitis_*bv 2\|*pneumoniae*\|HOT064)** | 0.001 | Health |
| ***Prevotella* (*histicola*\|*melaninogenica*)** | 0.003 | Health |
| ***Lactobacillus gasseri*** | 0.002 | Health |

Detailed analysis of the species over time showed a significant increase in health related bacteria .

## Claims

1. A composition comprising proteins and enzymes for use in promoting good oral health by increasing the health related bacterial flora of the mouth of the genus *Neisseria* and *Prevotella* and of the species *Neisseria flava, Neisseria (HOT499*|*sp_str_SMC_A9199), Neisseria sp._Oral_Taxon 020* and *Neisseria* sp._str._SMC_A9199, *Prevotella melaninogenica,* in which the proteins comprise lactoferrin and colostrum and in which the enzymes comprise amylase, an oxidase with oxygen as acceptor, haloperoxidase and a glycoside hydrolase.

2. A composition for use according to any preceding claim in which the glycoside hydrolase is lysozyme.

3. A composition for use according to any preceding claim in which the enzyme is selected from the group consisting of amylglucosidase, glucose oxidase, lactoperoxidase or mixtures thereof.

4. A composition for use according to any preceding claim in which the composition further comprises a non-ionic surfactant.

5. A composition for use according to claim 4 in which the non-ionic surfactant is a polyethylene glycol ethers of a fatty alcohol.

6. A composition for use according to claim 5 in which the non-ionic surfactant is steareth 30.

7. A composition for use according to any preceding claim in which the composition comprises a carrageenan based thickener.

8. A composition for use according to any preceding claim further comprising a zinc ion.

9. A composition for use according to any preceding claim which is in the form of a dentifrice.

## Patentansprüche

1. Proteine und Enzyme umfassende Zusammensetzung zur Verwendung bei der Steigerung guter Mundhygiene durch Zunahme der gesundheitsbezogenen Bakterienflora des Mundes der Gattung Neisseria und Prevotella und der Spezies Neisseria flava, Neisseria (HOT499|sp._str._SMC_A9199), Neisseria sp._Oral_Taxon_020 und Neisseria sp._str._SMC_A9199, Prevotella melaninogenica, wobei die Proteine umfassen Lactoferrin und Kolostrum und wobei die Enzyme Amylase, eine Oxidase mit Sauerstoff als Akzeptor, Haloperoxidase und eine Glycosidhydrolase umfassen.

2. Zusammensetzung zur Verwendung nach einem vorhergehenden Anspruch, wobei die Glycosidhydrolase Lysozym ist.

3. Zusammensetzung zur Verwendung nach einem vorhergehenden Anspruch, in welcher das Enzym aus der Gruppe ausgewählt ist, bestehend aus Amylglucosidase, Glucoseoxidase, Lactoperoxidase oder Mischungen davon.

4. Zusammensetzung zur Verwendung nach einem vorhergehenden Anspruch, in welcher die Zusammensetzung ferner ein nicht-ionisches Tensid umfasst.

5. Zusammensetzung zur Verwendung nach Anspruch 4, in welcher das nichtionische Tensid ein Polyethylenglycolether eines Fettalkohols ist.

6. Zusammensetzung zur Verwendung nach Anspruch 5, in welcher das nichtionische Tensid Steareth 30 ist.

7. Zusammensetzung zur Verwendung nach einem vorhergehenden Anspruch, in welcher die Zusammensetzung ein Verdickungsmittel auf Basis von Carrageenan umfasst.

8. Zusammensetzung zur Verwendung nach einem vorhergehenden Anspruch, ferner umfassend ein Zinkion.

9. Zusammensetzung zur Verwendung nach einem vorhergehenden Anspruch, die in Form eines Zahnputzmittels vorliegt.

## Revendications

1. Composition comprenant des protéines et enzymes pour une utilisation dans la promotion d'une bonne santé orale en augmentant la flore bactérienne de la bouche liée à la santé du genre *Neisseria* et *Prevotella* et des espèces *Neisseria flava, Neisseria (HOT499*|*sp._str._SMC_A9199), Neisseria sp._Oral_Taxon 020* et *Neisseria sp,_str,_SMC_A9199, Prevotella melaninogenica,* dans laquelle les protéines comprennent de la lactoferrine et du colostrum et dans laquelle les enzymes comprennent de l'amylase, une oxydase avec de l'oxygène comme accepteur, de l'haloperoxydase et une glycoside hydrolase.

2. Composition pour une utilisation selon la revendication précédente, dans laquelle la glycoside hydrolase est un lysozyme.

3. Composition pour une utilisation selon l'une quelconque des revendications précédentes, dans laquelle l'enzyme est choisie dans le groupe consistant en amylglucosidase, glucose oxydase, lactoperoxydase ou des mélanges de celles-ci.

4. Composition pour une utilisation selon l'une quelconque des revendications précédentes, dans laquelle la composition comprend de plus un tensioactif non-ionique.

5. Composition pour une utilisation selon la revendication 4, dans laquelle le tensioactif non-ionique est un éther de polyéthylèneglycol d'un alcool gras.

6. Composition pour une utilisation selon la revendication 5, dans laquelle le tensioactif non-ionique est stéareth 30.

7. Composition pour une utilisation selon l'une quelconque des revendications précédentes, dans laquelle la composition comprend un épaississant à base de carragheen.

8. Composition pour une utilisation selon l'une quelconque des revendications précédentes comprenant de plus un ion zinc.

9. Composition pour une utilisation selon l'une quelconque des revendications précédentes qui est dans la forme d'un dentifrice.
